# EUROPEAN PATENT SPECIFICATION

(11) **EP 2 114 442 B1**
(45) Date of publication and mention of the grant of the patent: **07.12.2016**
(21) Application number: 08706236.0
(22) Date of filing: 21.01.2008
(51) Int. Cl.: A61K 38/45, A61K 31/165, A61K 39/00, A61P 31/00, A61P 35/00, A61P 37/04

(54) **Histone H3 deacetylase inhibitor for use in the treatment of lung, prostate and cervical carcinomas**
Histone H3 Deacetylase Inhibitor für die Behandlung von Lungen-, Prostata- und Cervixkarzinoma
Inhibitor de la histone H3 déacétylase pour le traitement des carcinomes de poumouns, prostate et de cervix

(30) Priority: 19.01.2007 CA 2574531
(43) Date of publication of application: 11.11.2009
(73) Proprietor: biOmmune Technologies Inc., Vancouver, BC V6B K1 (CA)
(72) Inventor: SETIADI, Alverina, F., Sunnyvale, 94086 CA (US); DAVID, Muriel, D., 92296 Chatenay Malabry (FR); SEIPP, Robyn, P., Vancouver, BC V6R 1W4 (CA); HARTIKAINEN, Jennifer, Burnaby, BC V3N 4Y9 (CA); GOPAUL, Rayshad, Vancouver, BC V6P 4J4 (CA); JEFFERIES, Wilfred, A., Surrey, BC V4A 2V5 (CA)
(74) Representative: V.O.
(86) International application number: PCT/CA2008/000088
(87) International publication number: WO 2008/086612

(56) References cited:
- EP-A1- 1 174 438
- WO-A1-96/09380
- WO-A1-96/34952
- LOU ET AL: "Tumour immunity and T cell memory are induced by low dose inoculation with a non-replicating adenovirus encoding TAP1", VACCINE, ELSEVIER LTD, GB, vol. 25, no. 12, 12 December 2006 (2006-12-12), pages 2331-2339, XP005886453, ISSN: 0264-410X, DOI: 10.1016/J.VACCINE.2006.11.064
- Y LU ET AL: "Administration of subtumor regression dosage of TNF-[alpha] to mice with pre-existing parental tumors augments the vaccination effect of TNF gene-modified tumor through the induction of MHC class I molecule", GENE THERAPY, vol. 8, no. 7, 1 January 2001 (2001-01-01) , pages 499-507, XP055069742, DOI: 10.1038/sj.gt.3301429
- Y. NIE: "DNA hypermethylation is a mechanism for loss of expression of the HLA class I genes in human esophageal squamous cell carcinomas", CARCINOGENESIS, vol. 22, no. 10, 1 October 2001 (2001-10-01), pages 1615-1623, XP055069900, ISSN: 0143-3334, DOI: 10.1093/carcin/22.10.1615
- KOMATSU Y ET AL: "HISTONE DEACETYLASE INHIBITORS UP-REGULATE THE EXPRESSION OF CELL SURFACE MHC CLASS-I MOLECULES IN B16/BL6 CELLS", JOURNAL OF ANTIBIOTICS, JAPAN ANTIBIOTICS RESEARCH ASSOCIATION, TOKYO, JP, vol. 51, no. 1, 1 January 1998 (1998-01-01), pages 89-91, XP001055762, ISSN: 0021-8820
- KOMATSU YASUHIKO ET AL: "Cyclic hydroxamic-acid-containing peptide 31, a potent synthetic histone deacetylase inhibitor with antitumor activity", CANCER RESEARCH, AMERICAN ASSOCIATION FOR CANCER RESEARCH, US, vol. 61, no. 11, 1 June 2001 (2001-06-01), pages 4459-4466, XP002960287, ISSN: 0008-5472
- NAZMUL H KHAN A ET AL: "Histone deacetylase inhibitors induce TAP, LMP, Tapasin genes and MHC class I antigen presentation by melanoma cells", CANCER IMMUNOLOGY, IMMUNOTHERAPY, SPRINGER, BERLIN, DE, vol. 57, no. 5, 28 November 2007 (2007-11-28), pages 647-654, XP019586716, ISSN: 1432-0851
- WILLIAM J. MAGNER ET AL: "Activation of MHC Class I, II, and CD40 Gene Expression by Histone Deacetylase Inhibitors", J IMMUNUL, vol. 165, 1 January 2000 (2000-01-01), pages 7017-7024, XP055069969,
- A. F. SETIADI: "Identification of Mechanisms Underlying Transporter Associated with Antigen Processing Deficiency in Metastatic Murine Carcinomas", CANCER RESEARCH, vol. 65, no. 16, 15 August 2005 (2005-08-15), pages 7485-7492, XP055070285, ISSN: 0008-5472, DOI: 10.1158/0008-5472.CAN-03-3734
- DATABASE BIOSIS [Online] BIOSCIENCES INFORMATION SERVICE, PHILADELPHIA, PA, US; 16 November 2006 (2006-11-16), KATO NAOKO ET AL: "Induction of the NKG2D ligands expression by histone deacetylase inhibitor and its impact on the susceptibility of leukemic cells to the cytolytic activity of NKG2D-expressing cells.", XP002700412, Database accession no. PREV200700257672 & BLOOD, vol. 108, no. 11, Part 1, November 2006 (2006-11), pages 276A-277A, 48TH ANNUAL MEETING OF THE AMERICAN-SOCIETY-OF-HEMATOLOGY; ORLANDO, FL, USA; DECEMBER 09 -12, 2006 ISSN: 0006-4971
- HITOSHI ANDO ET AL: "Allelic variants of the human MHC class I chain-related B gene (MICB)", IMMUNOGENETICS, vol. 46, no. 6, 7 October 1997 (1997-10-07), pages 499-508, XP055070321, ISSN: 0093-7711, DOI: 10.1007/s002510050311
- SELIGER B ET AL: "ANALYSIS OF THE MAJOR HISTOCOMPATIBILITY COMPLEX CLASS I ANTIGEN PRESENTATION MACHINERY IN NORMAL AND MALIGNANT RENAL CELLS: EVIDENCE FOR DEFICIENCIES ASSOCIATED WITH TRANSFORMATION AND PROGRESSION", CANCER RESEARCH, AMERICAN ASSOCIATION FOR CANCER RESEARCH, US, vol. 56, 15 April 1996 (1996-04-15), pages 1756-1760, XP002011888, ISSN: 0008-5472
- MAGNER ET AL.: 'Apoptotic and necrotic cells induced by different agents vary in their expression of MHC and costimulatory genes' MOLECULAR IMMUNOLOGY vol. 42, no. 9, 23 November 2004, pages 1033 - 1042, XP008110257
- SMAHEL ET AL.: 'Immunisation with modified HPV16 E7 genes against mouse onocogenic TC-1 cell sublines with downregulated expression of MHC class I molecules' VACCINE vol. 21, no. 11-12, 07 March 2003, pages 1125 - 1136, XP004404289
- HAYASHI ET AL.: 'The mutation in the ATP-binding region of JAK1, identified in human uterine leiomyosarcomas, results in defective interferon-gamma inducibility of TAP1 and LMP2' ONCOGENE vol. 25, 13 February 2006, pages 4016 - 4026, XP003014052
- SETIADI ET AL.: 'Epigenetic Control of the Immune Escape Mechanisms in Malignant Carcinomas' MOLECULAR AND CELLULAR BIOLOGY vol. 27, no. 22, 17 September 2007, pages 7886 - 7894, XP008110261
- MANNING ET AL.: 'Induction of MHC class I molecule cell surface expression and epigenetic activation of antigen-processing machinery components in a murine model for human papilloma virus 16-associated tumours' IMMUNOLOGY vol. 123, no. 2, 28 August 2007, pages 218 - 227, XP008110260
- Shefali Agrawal ET AL: "Role of TAP-1 and/or TAP-2 antigen presentation defects in tumorigenicity of mouse melanoma", CELLULAR IMMUNOLOGY., vol. 228, no. 2, 1 April 2004 (2004-04-01) , pages 130-137, XP055238526, US ISSN: 0008-8749, DOI: 10.1016/j.cellimm.2004.04.006
- Tatsuaki Ishiguro ET AL: "Identification of Genes Differentially Expressed in B16 Murine Melanoma Sublines with Different Metastatic Potentials", Cancer Research, 15 February 1996 (1996-02-15), pages 875-879, XP055238532, UNITED STATES Retrieved from the Internet: URL:http://cancerres.aacrjournals.org/cont ent/56/4/875.full.pdf [retrieved on 2016-01-04]
- ZIMMER J ET AL: "Clinical and immunological aspects of HLA class I deficiency", QJM, vol. 98, no. 10, October 2005 (2005-10), pages 719-727, ISSN: 1460-2725
- GADOLA S D ET AL: "TAP deficiency syndrome", CLINICAL AND EXPERIMENTAL IMMUNOLOGY, vol. 121, no. 2, August 2000 (2000-08), pages 173-178, ISSN: 0009-9104
- YAN LIU ET AL: "Expression of antigen processing and presenting molecules in brain metastasis of breast cancer", CANCER IMMUNOLOGY, IMMUNOTHERAPY, SPRINGER, BERLIN, DE, vol. 61, no. 6, 8 November 2011 (2011-11-08), pages 789-801, XP035063182, ISSN: 1432-0851, DOI: 10.1007/S00262-011-1137-9
- SELIGER BARBARA ET AL: "Association of HLA class I antigen abnormalities with disease progression and early recurrence in prostate cancer", CANCER IMMUNOLOGY IMMUNOTHERAPY, vol. 59, no. 4, April 2010 (2010-04), pages 529-540, ISSN: 0340-7004
- BARBARA SELIGER ET AL: "Characterization of the Major Histocompatibility Complex Class I Deficiencies in B16 Melanoma Cells", CANCER RESEARCH, AMERICAN ASSOCIATION FOR CANCER RESEARCH, US, vol. 61, 1 February 2001 (2001-02-01), pages 1095-1099, XP007912856, ISSN: 0008-5472

## Description

### FIELD OF THE INVENTION

This invention relates to pharmaceutical compositions and uses thereof in medical treatments. More specifically it relates to a composition for use in the treatment of a mammal suffering from a disorder involving excess TAP-1 expression-deficient malignant cells which are selected from the group consisting of lung carcinoma cells, prostate carcinoma cells and cervical carcinoma cells, the composition comprising a TAP-1 expression increasing amount of a histone H3 deacetylase inhibitor that promotes transcription of a TAP-1 gene in the cells to cause enhanced MHC Class I surface expression of the cells, and a suitable adjuvant or carrier.

### BACKGROUND OF THE INVENTION

The cytotoxic T-lymphocyte (CTL) response is a major component of the immune system, active in immune surveillance and destnruction of infected or malignant cells and invading organisms expressing foreign antigens on their surface. The ligand of the antigen- specific T-cell receptor is a complex made up of a peptide fragment of a foreign antigen bound to major histocompatibility complex (MHC) molecules. Cytotoxic T lymphocytes recognize peptide bound to MHC Class 1 molecules, which are normally expressed at the cell surface as ternary complexes which include a peptide portion. Formation of the tennary complex involves transport into the lumen of the endoplasmic reticulum of peptides generated by protein degradation in the cytoplasm.

Two genes located in the MHC region have been identified and implicated in this transport of peptides from the cytoplasm, namely TAP-1 and TAP-2.

### BRIEF REFERENCE TO THE PRIOR ART

United States Patent 6.361.770 Jefferies et. al issued March 26, 2002, teaches a method of enhancing the expression of MHC Class 1 molecules on surfaces of target cells, by introducing into the target cell nucleic acid sequences encoding and expressing

TAP-1 or TAP-2. The expression in the target cells of TAP-1 or TAP-2 enhances the presentation of MHC Class I surface molecules on the target cells, so that they can be detected and eliminated by CTLs of the immune system. The method is particularly useful in connection with tumor cells which have a deficiency in proteasome components so that they have less than normal TAP expression and consequently do not express sufficient MHC Class I surface molecules to be recognized by CTLs. With in situ expression of augmented TAP from the added nucleic acid sequences, the target cells are brought under the recognition and action of the CTLs of the immune system.

Setiadi et al., (Cancer Res. 2005; 65, p. 7485 - 7492) described that TAP deficiency in many carcinomasis caused by a decrease in activity/expression of trans-acting factors regulating TAP-1 promoter activity, as well as decrease in TAP-1 mRNA stability.

It is known that regulation of chromatin structure plays an important role in controlling gene expression. Deregulation of genes involved in the modulation of chromatin stnzcture has been closely linked to uncontrolled cell growth, evasion of host immunosurveillance and development of tumors

### BRIEF SUMMARY OF THE INVENTION

It has now been found that chromatin remodeling plays a role in regulating the expression of transporters associated with antigen processing (TAP)-1, an important component of the antigen processing machinery. A high level of acetylated core histones in a chromatin template, particularly at the proximal region of an acetylation-sensitive promoter has previously been shown to associate with a transcriptionally active site, and so it has now been determined that histone H3 acetylation plays a significant role in the regulation of TAP-1 transcription. In particular, the highly specific histone deacetylase inhibitor trichostatin A has been found to be highly effective in promoting TAP-1 transcription and surface MHC Class I presentation in cancer cells, leading to their enhanced immunogenicity. We show that TAP- 2,Tapasin, MHC I, LMP2,7 etc are 11 upregulated by TSA.

The invention is as set forth in the independent claim. All other embodiments are described for illustrative purposes only, and any statement that they would also be part of the invention or other, similar statements, have to be seen in the context of the application as filed and do not alter the scope of the claims.

Thus the present invention refers to a pharmaceutical composition for use in the treatment of a mammal suffering from a disorder involving excess TAP-1 expression-deficient malignant cells which are selected from the group consisting of lung carcinoma cells, prostate carcinoma cells and cervical carcinoma cells, the composition comprising a TAP-1 expression increasing amount of a histone H3 deacetylase inhibitor that promotes transcription of a TAP-1 gene in the cells to cause enhanced MHC Class I surface expression of the cells, and a suitable adjuvant or carrier.

A process of enhancing the immunogenicity of cells is described by increasing the presentation of MHC Class I surface molecules for detection by cytotoxic T-lymphocyte cells which comprises administering to the cells an effective amount of a bio-acceptable substance that promotes transcription of genes in the cells to cause enhanced MHC Class I surface expression of the cells.

Further the preparation or manufacture of a composition for administration to a mammal suffering from a disorder involving excess TAP-1 expression-deficient cells is described, of a TAP-1 expression increasing amount of substance that promotes transcription of TAP-1 gene in the cells to cause enhanced MHC Class I surface expression of the cells and a suitable adjuvant or carrier.

### DESCRIPTION OF THE PREFERRED EMBODIMENTS

According to the invention, the bio-acceptable substance is a histone H3 deacetylase inhibitor, such as a hydroxamic acid-based histone H3 deacetylase inhibitor.

According to the invention,, the substance is trichostatin A.

While it is not intended that the present invention should be limited by any particular theory of action or biochemical mechanisms by which it is believed to operate, the following is offered and postulated for a better understanding of the invention and its practice.

The impairment of TAP-1 transcription in carcinomas is probably caused by a physical barrier resulting from a compact nucleosome structure around the TAP-1 promoter region, that prevents the access of general transcription factors and RNA polymerase II to the gene's promoter. As noted above, a high level of acetylated core histones in a chromatin template, particularly at the proximal region of an acetylation-sensitive promoter has been shown to associate with a transcriptionally active site. Raising the levels of histone H3 acetylation in TAP-1 promoter of several murine carcinoma cell lines showed that histone H3 acetylation plays a role in the regulation of TAP-1 transcription. Although histone H3 is not the only type of core histone that modifications were shown to play a role in expression of various genes, the correlation between the acetylation of histone H3 tails and activation of various genes has been widely studied and is now well established. TAP-2 and tapasin etc are also regulated this way as well.

The preferred histone deacetylase inhibitor (HDACi) for use in the present invention is the highly specific trichostatin A (TSA). TSA belongs to a group of hydroxamic acid-based histone deacetylase inhibitors that act selectively on genes, altering the transcription of only approximately 2% of expressed genes in cultured tumor cells and conferring anti-cancer effects *in vitro* and *in vivo.*

The process of the invention is of general application to mammalian cells exhibiting TAP-1 expression deficiencies, included malignant cells, virally infected cells and bacterially infected cells. Most preferred is use in enhancing TAP-1 expression in malignant tumor cells, which are lung carcinoma cells, prostate carcinoma cells and cervical carcinoma cells. Since carcinoma cells express the papiloma E6 and E7 genes, the enhancement of recognition of papiloma virus antigens is enabled by the present invention. Since Papillomas are herpes viruses, this approach predictably works for all herpes viruses.

Compositions according to the invention may be administered to patients by any of a variety of routes, provided that they reach the site(s) of the TAP expression deficient cells. Such routes include intravenous, intramuscular, intraperitoneal, oral, nasal, parenteral, Inter tumoural also ex vivo by treating tumours or dendritic cells with said compound and then reintroducing them into the patient etc. Appropriate doses are determined by the condition of the patient and degree of severity of the disorder under treatment, but are within the ordinary skill of the attending clinician based upon analogy with other malignancy treating pharmaceuticals.

A particularly preferred use of the compositions of the present invention is as adjuvants in connection with known cancer, bacterial infection, protozoan and viral infection treatments. When the compositions of the invention are used as adjuvants with other pharmaceutically effective treatments such as vaccines, a many-fold reduction in the amount of vaccine for effectiveness is to be expected.

### Brief reference to the drawings

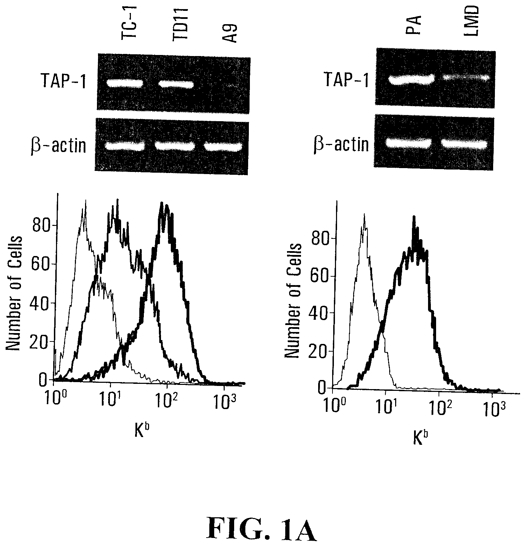

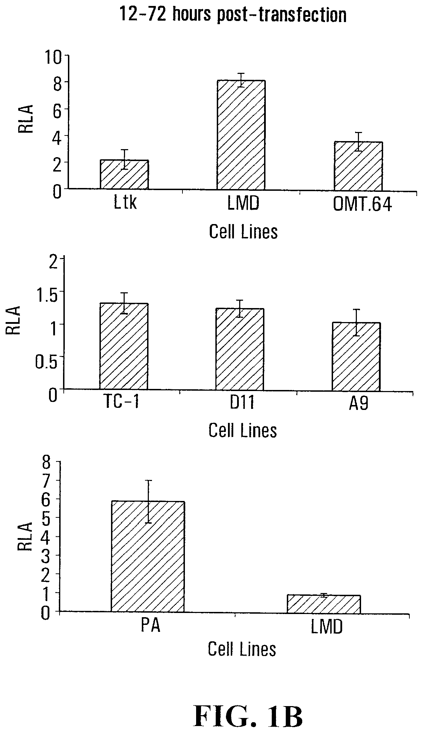

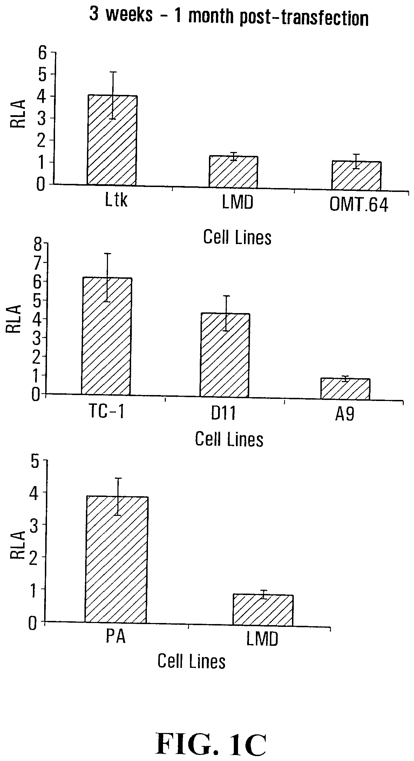

The accompanying Figures 1- 8 of drawings are graphical and pictorial representations of the results obtained from the various specific experimental Examples described and discussed below in some detail.

The invention is further described in the following "Experimental Procedures" and "Examples" sections.

### Experimental Procedures

### Cell lines and reagents

In this study, 3 cell lines: TC-1, TC-I/DI1 and TC-1/A9 were used as HPV- positive carcinoma models. The TC-1 cell line was developed from transformation of murine primary lung cells with HPV 16 E6 and E7 oncogenes and activated H-ras [1] . TC-1 /D 11 and TC-1 /A9 are clones of TC-1 cells with downregulated expression of TAP-1 and MHC class I[2]. A murine primary prostate cancer cell line, PA, and its metastatic, TAP- and MHC class I-deficient derivative, LMD were also used [3].

The TC-1 cell line that was developed from transformation of C57BL/6 primary lung cells with HPV16 E6 and E7 oncogenes and activated H-ras was provided by Dr. T.C. Wu, Johns Hopkins University, Baltimore, MD [7]. The TC-1/D11 and TC-1/A9 cell lines were provided by Dr. M. Smahel, Institute of Hematology and Blood Transfusion, Prague, Czech Republic [12]. The TC-1/D11 and TC-1/A9 are abbreviated to D11 and A9, respectively, in this paper. The CMT.64 cell line was established from a spontaneous lung carcinoma of a C57BL/6 mouse [4]. The Ltk (=L-M(TK-)) fibroblast cell line was derived from a C3H/An mouse (ATCC, Manassas, VA). All cell lines above were grown in DMEM media. The PA and LMD cell lines, derived from primary and metastatic prostate carcinoma of a 129/Sv mouse, respectively (a kind gift of Dr. T. C. Thompson, Baylor College of Medicine, Houston, TX) [3], as well as the B 16F10 (B 16) melanoma [5] and RMA lymphoma [6] cell lines, both derived from C57BL/6 mice, were maintained in RPMI 1640 media. RPMI 1640 and DMEM media were supplemented with 10% heat-inactivated fetal bovine serum (FBS), 2 mM L-glutamine, 100 U/ml penicillin, 100 g/ml streptomycin, and 10 mM HEPES. One millimolar sodium pyruvate and 0.4 mg/mi G418 were also added to the

DMEM media for the TC-1, D11 and A9 cells. Cells were untreated or treated with 100 ng/ml TSA (Sigma, St. Louis, MO) for 24 hours (CMT.64, B 16, PA and LMD) or 48 hours (TC- 1, D 11 and A9), or with 50 ng/ml IFN-y for 48 hours.

### Reverse transcription-PCR analysis.

Primers used for PCR amplifications (Sigma-Genosys, Oakville, ON and Integrated DNA Technologies (IDT), Coralville, IA) are listed in Table 1. Total cellular RNA were extracted using Trizol Reagent (Invitrogen, Burlington, ON) and contaminating DNA was removed by treating the RNA samples with DNase 1(Ambion Inc., Austin, TX). Reverse transcription of 1 g of total cellular RNA was performed using the reverse transcription kit from Invitrogen, in a total volume of 20 µl. 2 µl aliquots of cDNA were used as a template for PCR in a total of 50 µl reaction mixture containing 1 x PCR buffer, 250 M deoxynucleotide triphosphate, 1.5 mM MgCl₂, 0.2 µM of each primer and 2.5 units Taq or Platinum Taq DNA Polymerase. All PCR reagents were obtained from Invitrogen and Fermentas, Burlington, ON. cDNA amplifications were carried out with specific primer sets in a T-gradient thermocycler (Biometra, Goettingen, Germany) with 25-35 cycles of denaturation (1 min, 95°C), annealing (1 min, 54-64°C), and elongation (2 min, 72°C). The cycling was concluded with a final extension at 72°C for 10 min. Twenty microliters of amplified products were analyzed on agarose gels, stained with ethidium bromide and photographed under UV light.

### Real-time quantitative PCR analysis.

In this study, this method was employed for quantification of levels of endogenous TAP-1 promoter co-precipitating with antibodies in the chromatin immunoprecipitation assays and quantification of copy number of the pTAP1-luc construct integrated in stably transfected cells. Purified genomic DNAs were used as templates for amplifications using 200-500 nM of each primer and 1 µl SYBR Green

Taq ReadyMix (Roche) in a total of 10 µl reaction mixture. 37 cycles of denaturation (5 seconds, 95°C), annealing (5 seconds, 61-63 °C), and elongation (20 seconds, 72 °C) were performed using a Roche LightCycler.

### Chromatin immunoprecipitation assays.

Chromatin immunoprecipitation experiments using 7 x 10⁶ cells per sample were done as previously described [7]. Five micrograms of anti-RNA pol II (N-20, sc-899, Santa Cruz Biotechnologies, Santa Cruz, CA), anti-acetyl-histone H3 (Upstate Biotechnology Inc., Lake Placid, NY) or anti-CBP (A-22, sc-369, Santa Cruz) polyclonal antibody (Ab) were used for the immunoprecipitation. Levels of murine TAP-1 promoter or co-immunoprecipitating with the antibody from each sample were quantified by real-time PCR using primers specific for the TAP-1 promoter. Primers specific to the 3'-end of TAP-1 promoter were used for PCR if the templates were immunoprecipitated using anti-RNA po1 II or anti-acetyl-histone H3 antibody, while 5'-end-specific primers were used for templates immunoprecipitated using anti-CBP antibody. Serial dilutions of plasmid containing the murine TAP-1 promoter were used to generate a standard curve using the TAP-1 promoter-specific primer set.

### Plasmids construction

A plasmid containing an EGFP gene driven by TAP-1 promoter (pTAP1-EGFP) was constructed as described previously [8]. A similar construct containing a luciferase gene driven by TAP-1 promoter (pTAP 1-luc) was created by inserting the TAP-1 promoter between the Sac I and Bgl II sites of pGL4.14[luc2/Hygro] vector (Promega, Madison, WI). Several truncated TAP-1 promoter constructs were also made by cloning its 5'-end truncations into pGL4.I4[luc2/Hygro] vector. Primers used for PCR amplifications of the full TAP-1 promoter and its truncations are listed in Table 1.

### Transfection and selection

TC-1, D11, A9, PA and LMD cells were transfected with the TAP-1 promoter constructs or the promoterless pGL4.14[luc2/Hygro] vector using ExGen 500 in vitro Transfection Reagent (Fermentas). Transient transfectants were analysed between 12-72 hours after transfection. To obtain stable transfectants, the transfected cells were selected for 3 weeks in the presence of the following concentrations of Hygromycin B (Sigma): 550 ng/ml for TC-1 cells, 250 ng/ml for D11, A9 and LMD cells, 200 ng/ml for PA cells.

### Luciferase assays.

Relative luciferase activity (RLA) in transient transfectants was assessed by dual luciferase assay (Promega) within 12-72 hours after transfection. RLA in stable transfectants (3 weeks -1 month post-transfection) was assessed by Bright-Glo luciferase assay (Promega) and determined by subtracting the results with corresponding values obtained from transfection with promoterless pGL4.14[luc2/Hygro] vector alone and by further normalizing the values with copy number of plasmids integrated into the genome of each stable transfectant.

### Western Blot.

Fifty micrograms of proteins per sample were separated through 6% (p300, CBP and TAP-1) or 15 %((3-actin and acetyl-histone H3) SDS-PAGE. Proteins were transferred to a nitrocellulose membrane (Bio-rad, Hercules, CA). Blots were blocked with 5% skim milk in PBS and incubated with appropriate Ab dilutions. The following rabbit polyclonal Abs were used in these studies: anti-mouse TAP-1 (made by Linda Li in Jefferies Lab); anti- acetyl-histone H3 (Upstate Biotechnology Inc.); anti-p300 (C-20, sc-585, Santa Cruz); anti-CBP (A-22, sc-369, Santa Cruz). Secondary Ab used was HRP-conjugated goat anti-rabbit secondary Ab (Jackson Immunoresearch Lab., West Grove, PA). For the loading controls, anti β-actin mouse monoclonal Ab (Sigma) was used, followed by HRP-conjugated goat anti-mouse secondary Ab (Pierce, Rockford, IL). Blots were developed using Lumi-light reagents (Pierce).

### Cytotoxicity assays.

CTL effector cells were generated by injecting C57B16 mice with 1* 107 TCIP of Vesicular Stomatitis Virus (VSV). The spleens were collected 7 days later, homogenized and incubated for 5 days in CTL medium (RPMI-1640 containing 10% FBS (Hyclone), 20 mM HEPES, 1% NEAA, 1% sodium pyruvate, 1% L-glutamine, 1% penicillin/streptomycin and 0.1 % 2-ME) with luM VSV-NP peptide (RGYVYQGL). TC-1, D11 and A9 cells were treated with IFN-y (50 ng/ml) for 48 hours, TSA (100 ng/ml) for 24 hours or left untreated prior to infection with VSV at an MOI of 7.5 for 16 hours. Cells were washed with PBS and loaded with 51Cr by incubating 106 cells with 100 Ci of 51Cr (as sodium chromate; Amersham, Arlington Heights, IL) in 250 I of CTL medium for 1 hour. Following three washes with PBS, the target cells were incubated with the effector cells at the indicated ratios for 4 hr. 100 μl of supernatant from each well were collected and the ⁵¹Cr release was measured by a y-counter (LKB Instraments, Gaithersburg, MD). The specific ⁵¹Cr release was calculated as follows: ((experimental - media control) / (total - media control)) x 100%. The total release was obtained by lysis of the cells with a 5% Triton X-100 solution.

### Establishment of HPV-positive cancer xenografts and treatment with Trichostatin A.

A total of 3x10⁵ cells of TC-1 or A9 in PBS were injected subcutaneously into seven-week-old female C57BL/6 syngeneic mice (Charles River, St. Constant, QC). Mice
were assigned to 3 groups of 4 animals. TSA was dissolved in DMSO to a concentration of 0.2 mg/ml. Daily treatment with 50 µl TSA (500 g/kg) or DMSO (vehicle control) was administered via intraperitoneal injection for 20 days, starting on day 7 after injection with tumor cells. Mice were weighed weekly, and their behavior and food intake were monitored throughout the course of the experiment. Tumors were measured 3 times a week and tumor volume was calculated using the formula: tumor volume =length x width x height x n/6 [32]. The study period was determined by the size of the tumors in the A9 group treated with DMSO vehicle control.

### Experimental Results:

### Example 1.

Using the reagents and procedures described above, it was demonstrated that EP-1 mRNA expression in mouse prostate and HPV-positive carcinoma models correlates with their surface MHC class I expression.

It was confirmed that the TAP-1 expression levels correlate with the MHC class I surface expression levels in both groups of cell lines used (mouse prostate carcinoma and HPV-positive carcinoma models) These results are shown on Fig. 1. Luciferase gene expression that is controlled by TAP-1 promoter generally matches the endogenous TAP-1 levels after the transfectants become stable. Figure 1A shows analysis of TAP- 1 and surface MHC class I expression by RT-PCR and flow cytometry, respectively. Shaded area, thin and thick lines represent low (A9 or LMD), medium (D11) and high (TC-1 or PA) levels of MHC class I expression, respectively. Amplification of 0-actin cDNA served as an internal control in the RT- PCR analysis. Data are representatives of three experiments. Figure 1B - relative luciferase activity (RLA) in transient transfectants was assessed by dual luciferase assay within 12-72 hours after transfection. RLA in stable transfectants (3 weeks - 1 month post-transfection) was determined by subtracting the results with corresponding values
obtained from transfection with promoterless pGL4.14[luc2/Hygro] vector alone and by further normalizing the values with copy number of plasmids integrated into the genome of each stable transfectant. The smallest value was arbitrarily determined as 1. Columns, average of four to six experiments; bars, SEM.In the prostate carcinoma model, PA cells that expressed a higher level of surface MHC class I than LMD cells also expressed a higher level of TAP-1. Similarly, in the HPV-positive carcinoma model, TC-1, D11 and A9 cells that express high, moderate and low levels of surface MHC class I, respectively, also expressed TAP-1 in the same order as the MHC class I levels.

### Example 2.

The role of chromatin remodeling in the regulation of TAP-1 transcription was investigated.

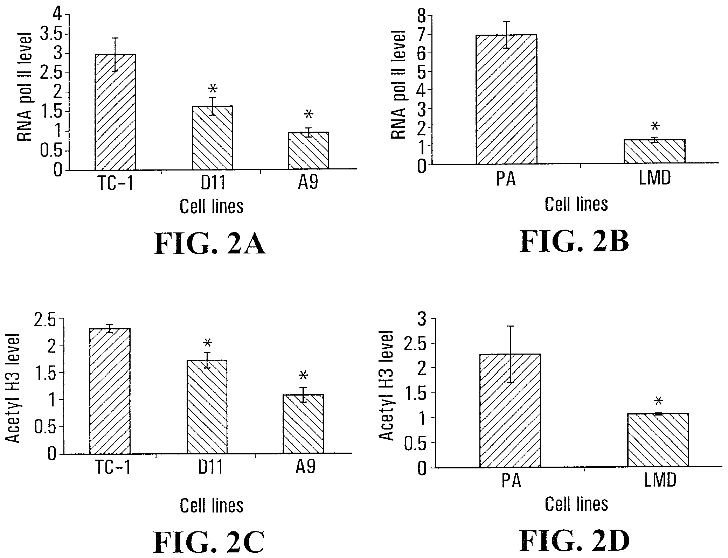

Previous observations by fluorescence microscopy and flow cytometry showed that a few days after transfection of several groups of TAP-expressing and TAP-deficient cell lines with a reporter construct containing an EGFP gene driven by TAP-1 promoter, many cells in the TAP- deficient groups expressed similar or even higher levels of EGFP than most cells in the TAP- expressing groups did (data not shown), while the EGFP expression levels in stable transfectants matched the endogenous TAP-1 expression profiles (Setiadi et.al., Cancer Res 65, 7485-7492). In this experiment, there was generated a Luciferase reporter construct by cloning the mouse TAP-1 promoter upstream of the luc gene in the pGL4.14[luc2/Hygro] vector (pTAPI-luc) and the construct was transfected into the cell lines. As in previous observations of EGFP levels, the levels of luc gene expression in stable transfectants were found to match the endogenous TAP-1 expression profiles better than those in transient transfectants, with the exception of the prostate carcinoma model that showed equally matching profiles of both the transient and the stable transfectants to the endogenous TAP-1 expression. This is shown on Figure 1B. In addition, RNA
polymerase II (pol II) binding to TAP-1 promoter in cells with low TAP-1 expression levels was relatively lower than that in cells with higher TAP-1 expression levels, as shown on Figure 2. The levels of RNA Pol II or acetyl-histone H3 in TAP-1 promoter of each cell line were assessed by chromatin immunoprecipitation using anti-RNA pol II or anti- acetyl-histone H3 antibody, respectively. The eluted DNA fragment were purified and used as templates for real-time PCR analysis using primers specific for the 3'-end of the TAP-1 promoter. Relative RNA po1 II or acetyl-histone H3 levels were determined as the ratio of copy numbers of the eluted TAP-1 promoter and copy numbers of the corresponding inputs. The smallest ratio was arbitrarily determined as 1. Columns, average of three to six experiments; bars, SEM. * P <.05 compared with cells that expressed higher MHC class I in the same assay.

These results indicate the existence of a physical barrier that lowers the access of general transcription factors and RNA pol II to TAP-1 promoter, as the promoter has integrated into the genome. The LMD cells may have additional defects in factors unrelated to chromatin remodeling that impair the transcription of genes driven by the TAP-1 promoter.

### Example 3 Demonstration that histone H3 acetylation is low in TAP-1 promoter of MHC class I-deficient carcinomas.

This experiment investigated the levels of histone H3 acetylation in TAP-1 promoter of the murine prostate and the cervical carcinoma cell lines in order to determine the role of histone H3 acetylation in the regulation of TAP-1 transcription. Although histone H3 is not the only type of core histones that modifications have been shown elsewhere to play a role in various genes expression, this experiment investigated its acetylation states, since the correlation between the acetylation of histone H3 tails and various gene activation has been widely studied and is now well established [9, 10, 11].

In accordance with the levels of RNA pol II in TAP-1 promoter and of TAP-1 transcription (Figure 1 and 2), the levels of acetyl-histone H3 were found to be lower in TAP-1 promoter of cells that express lower levels of TAP-1 (Figure 2). In the HPV- positive carcinoma model, D11 cells that express moderate level of TAP-1 compare to the TC-1 and A9 consequently were found to have moderate level of acetyl-histone H3 in the TAP-1 promoter. In the prostate carcinoma model, the metastatic, TAP-deficient LMD cells also have less acetyl- histone H3 binding to the TAP-1 promoter than in the primary cells, PA. Acetyl- histone H3 levels in TAP-1 promoter of several other TAP-expressing (Ltk and RMA) and TAP- deficient (CMT.64 and B 16) cell lines were also tested, and higher levels of acetyl- histone H3 levels in Ltk and RMA than in CMT.64 and B 16 (data not shown) were found.

### Example 4- Effects of trichostatin A (TSA), a histone deacetylase inhibitor treatment in the expression of TAP-1 and other antigen processing machinery (APM) components.

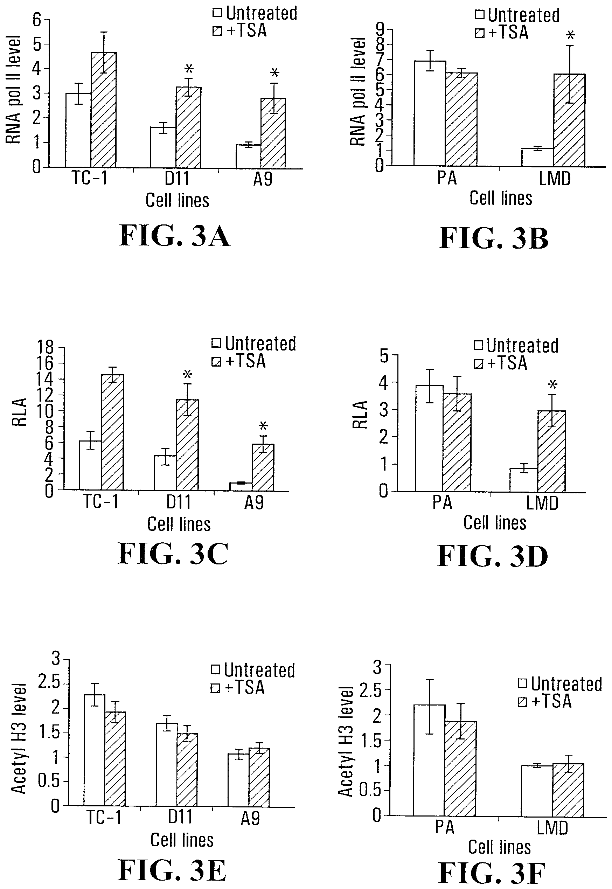

As the low level of histone H3 acetylation in TAP-1 promoter seems to contribute to TAP-1 deficiency in carcinomas, a further investigation was conducted to determine whether treatment of TAP-deficient carcinoma cells with TSA would increase the TAP-1 expression in the cells. The results are presented on Fig. 3. The levels of RNA Pol II or acetyl- histone H3 in TAP-1 promoter of each cell line were assessed by chromatin immunoprecipitation using anti-RNA pol II or anti-acetyl-histone H3 antibody, respectively. The eluted DNA fragments were purified and used as templates for real-time PCR analysis using primers specific for the 3'- end of the TAP-1 promoter. Relative RNA pol II or acetyl-histone H3 levels were determined as the ratio of copy
numbers of the eluted TAP-1 promoter and copy numbers of the corresponding inputs. The smallest ratio was arbitrarily determined as 1. Columns, average of three to six experiments; bars, SEM. * P<.05 compared with cells that expressed higher MHC class I in the same assay.

Chromatin immunoprecipitation results in figure 3 showed that TSA treatment enhanced the recruitment of RNA pol II complex to the TAP-1 promoter in most cell lines, particularly in the TAP-deficient cells. In both the HPV-positive and prostate carcinoma models, TSA treatment increased the level of RNA pol II binding to TAP-1 promoter of TAP-deficient cells to similar levels as in TAP-expressing cells.

In parallel to the increase of RNA pol II binding to the TAP-1 promoter that is known to be an important event to initiate a transcription process, TAP-1 promoter activity, measured based on luc expression in cells stably transfected with pTAP1-luc construct, had also increased significantly in TAP-deficient cells upon treatment with TSA (Figure 3). However, chromatin immunoprecipitation analysis showed that TSA treatment did not significantly alter the levels of
acetyl-histone H3 in TAP-1 promoter of all cell lines tested (Figure 3). This suggests that the TSA action that enhanced the TAP-1 promoter activity did not occur due to direct improvement of acetyl-histone H3 levels in the TAP-1 promoter itself.

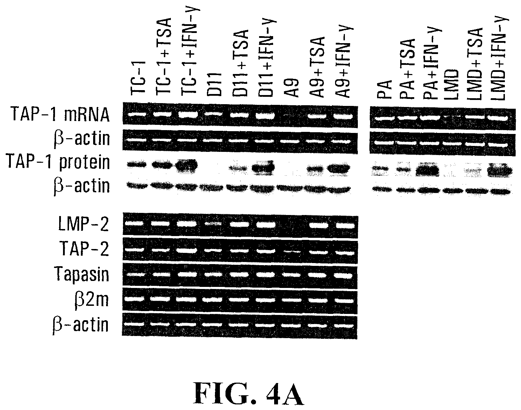

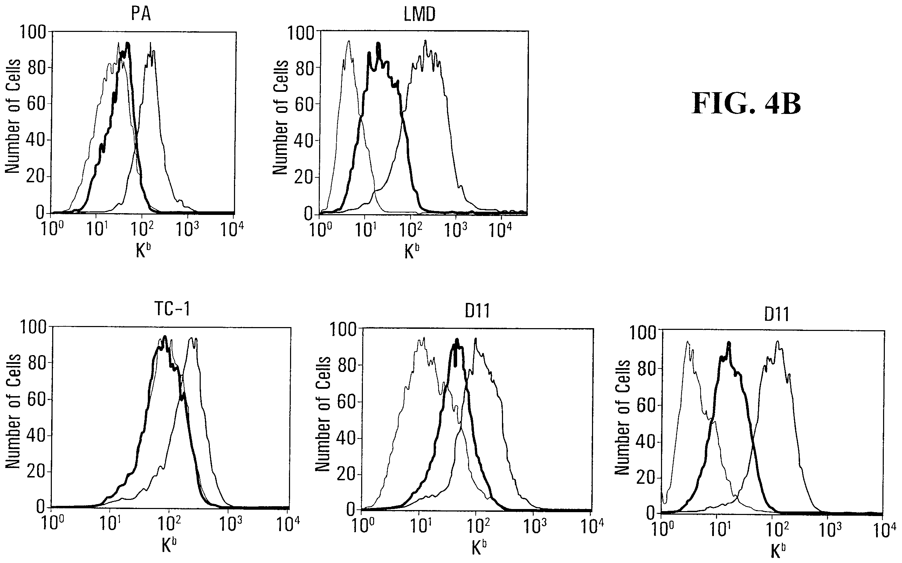

Figure 4 of the accompanying drawings shows efficacy of TSA treatment in MHC class I antigen presentation.

A, Expression of TAP-1 and several other APM components in all cell lines, particularly in the TAP-deficient carcinomas, was up-regulated with TSA treatment. Amplification of APM cDNA from IFN-γ-treated cells was used as a positive control. β-actin expression served as a loading control. Data are representatives of three experiments.

*B*, Surface H-2K^{b} expression, particularly on MHC class I-deficient cells, was enhanced by TSA treatment. Cells untreated (shaded areas) or treated with 100 ng/ml TSA (thick lines) or 50 ng/ml IFN-y (thin lines) were stained with PE-conjugated anti-H-2Kb mAb. Data are representatives of three experiments.

RT-PCR and Westem Blot analysis also showed that the expression of TAP-1 was up- regulated upon TSA treatment (Figure 4A). Additional RT-PCR analysis of several other APM components in the HPV-positive carcinoma cell lines also demonstrated an up- regulation of
expression of LMP-2, TAP-2 and tapasin, particularly in the TAP-deficient cells upon TSA treatment (Figure 4A). It was also observed that the LMP-2 and TAP-2 genes have similar patterns of expression as the TAP-1 gene in TC-1, D11 and A9 cells (high, moderate and low, respectively), and are also inducible by TSA and IFN-y (Figure 4A).

Western blot analysis of TAP-1 expression was performed using lysate of cells that was pre-treated with 20 to 200 ng/ml TSA for 24 to 48 hours, and representative data from cells treated with 100 ng/ml TSA for 24 hours (PA and LMD) or 48 hours (TC-1, D 11 and A9) are shown in figure 4A. The analysis showed increasing levels of TAP-1 expression in parallel with the increase of acetyl-histone H3 accumulation in the whole cell lysates of TAP-deficient cells upon increasing dose of treatment with TSA (data not shown). Optimal dose and period of treatment that resulted in an optimal induction of TAP-1 expression in LMD, CMT.64 and B 16 were 100 ng/ml for 24 hours, while 48 hours of treatment with the same dose of TSA were needed for the D11 and A9 cells.

### Example 5

This experiment demonstrated that TSA treatment increases surface MHC class I expression and CTL killing of the cancer cells.

Since the expression of several antigen processing components increases upon TSA treatment, it was investigated whether the treatment would further increase the level of surface MHC class I expression in the carcinoma cell lines. This would potentially enhance tumor antigen presentation and subsequent killing of the cancer cells by CTLs. Flow cytometric analysis showed that TSA treatment increased surface expression H-2Kb by approximately 10 folds in MHC class I-deficient cells, whereas the levels remained the same in PA and TC- 1 cells, that had originally expressed high levels of surface H-2K^{b} (Figure 4B). IFN-y treatment increased the surface H-2Kb expression in all cell lines. Similar induction of MHC class I expression by TSA was also observed in TAP-deficient lung carcinoma (CMT.64) and melanoma (B 16, not part of the invention) (data not shown).

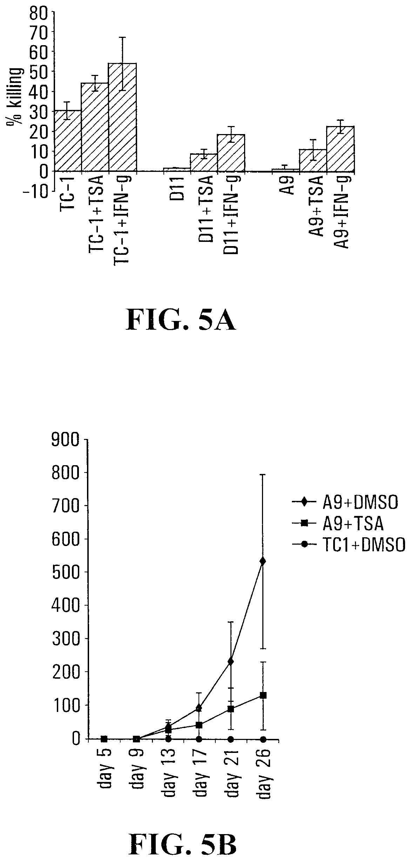

The fact that TSA treatment enhances killing of tumor cells by CTL and suppresses tumor growth in vivo is shown on accompanying Figure 5.

A, CTL recognition of uninfected or VSV-infected TC- 1, D11 and A9 cells, untreated or treated with TSA or IFN-y for 48 hours before infection with VSV. All cells were infected with VSV at a MOI of 7.5 for 16hr. *Columns,* average of three experiments; *bars,* SEM.

*B*, A9 (MHC class I-deficient cells) tumor growth was suppressed in mice treated with 500 g/kg of TSA daily compared to in those treated with DMSO vehicle control (n = 4 per treatment group). TC-1 group represented tumor growth in mice injected with high MHC class I- expressing cells (n =4). Data represent the mean tumor volume SEM.

Furthermore, CTL assays were performed in order to test whether the enhanced level of surface H-2K^{b} expression in cells after TSA treatment would subsequently increase the recognition and the killing of VSV-infected cancer cells by VSV-specific cytotoxic T lymphocytes. Peptides derived from the VSV infection of target cells could be presented in the context of K^{b}. CTL assays were performed using
effector:target ratio of 0.8:1 to 200:1, and representative data from 22:1 effector:target ratio using TC-1, D11 and A9 as target cells are shown in Figure 5A. The results showed that D11 and A9 cells, that expressed lower surface K^{b} than the TC-1 did, were Icilled less by the CTLs. TSA treatment for 24 hours prior to VSV infection of the cancer cells enhanced CTL killing of the virus-infected TC-1, D11 and A9 cells by approximately 1.4, 6 and 7 folds, respectively. IFN-y treatment resulted in maximal induction of the level of killing of all VSV-infected cell lines. Similar trends were also observed in CTL assays using VSV-infected CMT.64 and B16 as target cells (data not shown). TSA treatment of CMT.64 and B16 cells 24 hours prior to VSV infection enhanced the levels of killing by 5 and 20 folds, respectively. LMD cells remained resistant to CTL killing despite induction of APM and MHC class I expression by IFN-y due to an unknown mechanism independent of MHC class I expression (see Lee, H.M. et.al, Cancer Res. 60, 1927-33).

### Example 6- TSA treatment suppresses tumor growth in vivo.

TC-1 and A9 cells were cultured in vitro and were passaged less than 8 times before 3x105 of each group of the cells were injected subcutaneously into 7-week-old female C57BL/6 syngeneic mice. Daily treatment with TSA or DMSO vehicle control began on day 7 after cells injection, as the animals started to grow palpable A9 tumors. The dose of 500 g/kg TSA per animal per day was chosen as it had been successfully used by others to suppress other types of tumor growth in murine tumor models - see for example Canes, D et.al, Int J Cancer 113, 841- 848 In this study, it was observed that tumor growth was slower in mice that received daily treatment with TSA compare to the DMSO control group (Figure 5B). In addition, TC-1 cells that expressed high levels of MHC class I on the surface, were found to be significantly less tumorigenic than the A9 cells (Figure 5B). TC-1 tumors started to grow at approximately 3 weeks - 1 month after s.c. injection of mice with 3x10⁵ to
4x10⁵ tumor cells. No tumor was detected beyond 1 month after injection with less than 3x105 TC-1 tumor cells (data not shown). These observations matched the in vitro findings that demonstrated that the increase of expression of APM components and MHC class I antigen presentation correlate with higher killing of the cancer cells, thus suppression of tumor growth.

### Example 7 (provides technical information)- Mechanism of TAP-1 induction by IFN-y.

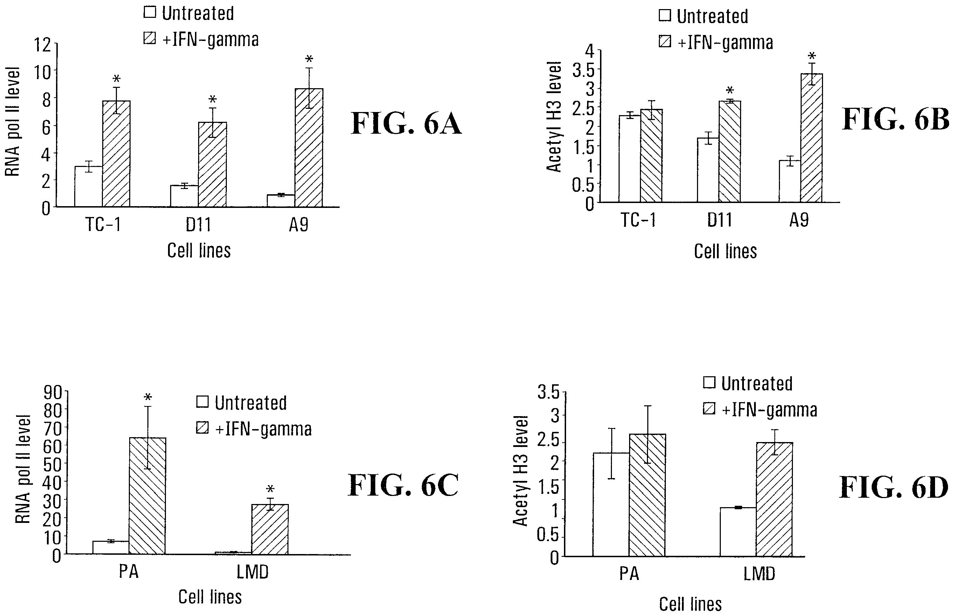

IFN-y is known as a potent inducer of TAP-1 and surface MHC class I expression in cancer cells [Setiadi,A.F et.al, op.cit.); however, little is known about molecular mechanisms that lead to TAP-1 induction by IFN-y. In this study, it was found that IFN-y treatment increased the level of acetyl-histone H3 and RNA pol II in TAP-1 promoter. Figure 6 of the accompanying drawings also shows chromatin immunoprecipitation using anti-RNA pol II or anti-acetyl- histone H3 antibody, performed as described earlier. *Columns,* average of three experiments; *bars,* SEM. * P <.05 compared with untreated cells.

Previous studies proposed that transcriptional activators that increased the recruitment of RNA pol II complex to a promoter should in parallel increase histone acetylation in the promoter region (Struhl, K., Genes Dev 12, 599-606. The results presented here suggest that one possible mechanism of TAP-1 induction by IFN-y is via the improvement of histone H3 acetylation that relaxes the chromosome structure around the TAP-1 promoter region, thus increases the accessibility of general transcription factors and RNA pol II complex to the TAP-1 promoter.

### Example 8 - Identification of regions in TAP-1 promoter responsible for differential activity of the promoter in TAP-expressing and TAP-deficient cells.

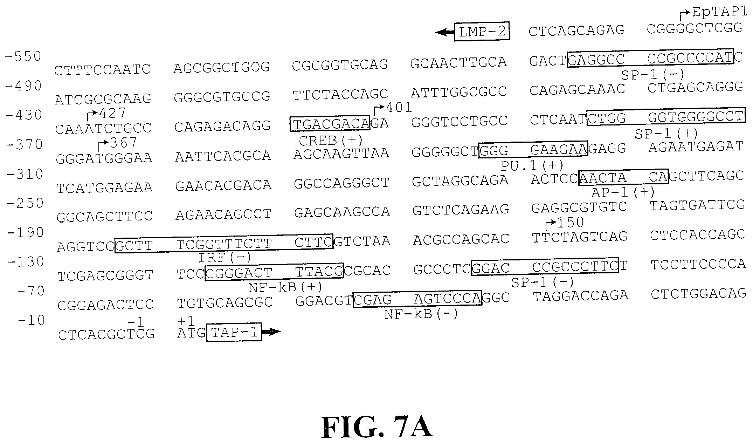

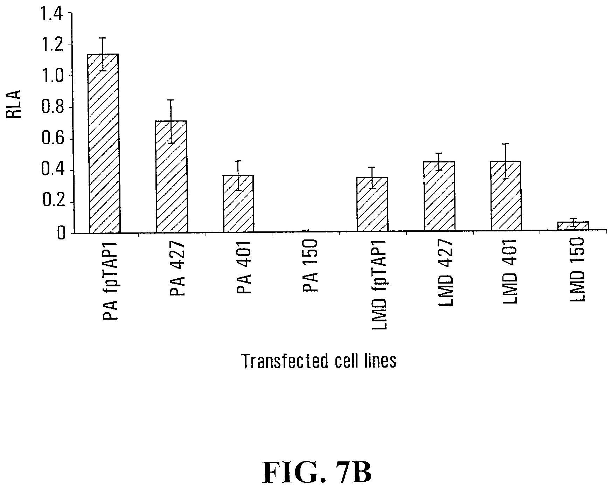

Several TAP-1 promoter constructs were made by cloning full TAP-1 promoter (fpTAPI) or its 5'-end truncations (427, 401 and 150) into pGL4.14[luc2/Hygro] vector. Figure 7 of the accompanying drawings shows analysis of TAP-i promoter region that is responsible for differential activity in TAP-expressing and TAP-deficient cells.

A, TAP-1 promoter sequence with transcription factor binding motifs and 5' truncation sites. The TAP-1 ATG codon was arbitrarily determined as +1. Motifs located on the sense strand are indicated by a(+) and motifs located on the antisense strand are indicated by a (-).

*B,* Relative activity of Luciferase driven by full and truncated TAP-1 promoter in PA and LMD cell lines. RLA in the stable transfectants was determined as described previously. The largest value was arbitrarily determined as 1. Columns, average of four experiments; bars, SEM.

The ATG codon of TAP-1 gene was arbitrarily numbered as +1, and the truncated promoters were named based on the starting base position of forward primers with respect to the ATG codon (Figure 7A). Stable transfectants of PA and LMD cells (TAP- expressing and TAP- deficient, respectively) were obtained after selecting the transfected cells in Hygromycin B for 3 weeks. Luciferase assay was then performed using 10,000 cells from each stable transfectants. It was found that as approximately 160 base pairs of nucleotides were truncated from the 5'-end of TAP-1 promoter (construct 401), the Luciferase expression in PA cells dropped more than 3 folds, to the same level as the Luciferase driven by a full TAP-1 promoter in LMD cells (Figure 7B). Final truncation to "150" resulted in almost total loss of activity of the promoter (Figure 7B). These results indicate that the lack of transcription activator(s) binding to LMD cells is likely to fall within base #-557 and approximately #-401 of the TAP-1 promoter.

### Example 9 - Analysis of CREB-binding protein (CBP) expression and its association with TAP-1 promoter.

Since the results in this study indicate that histone acetylation plays a role in the regulation of TAP-1 expression, it was further investigated whether transcriptional activators/co- activators of TAP-1 that are deficient or non-functional in carcinomas are those with intrinsic histone acetyl transferase (HAT) activity. The functionality of one of the well-known transcription co-activators that possess intrinsic HAT activity was analyzed, namely the cyclic AMP-responsive element binding (CREB)-binding protein (CBP), since CREB binding site was found within the TAP-1 promoter region that was shown to be responsible for differential activity of the promoter in TAP-expressing and TAP-deficient cells, specifically in-between base #-427 and #-401 (Figure 7A and 7B). In addition, CBP was known to acetylate histone H3 and H4, and the HAT activity and recruitment of CBP were shown to be stimulated by various transcription factors (Legube, G. et.al., EMBO Rep 4, 944-47) including SP-1 and AP-1 that binding sites are also present in the TAP-1 promoter (Figure 7A).

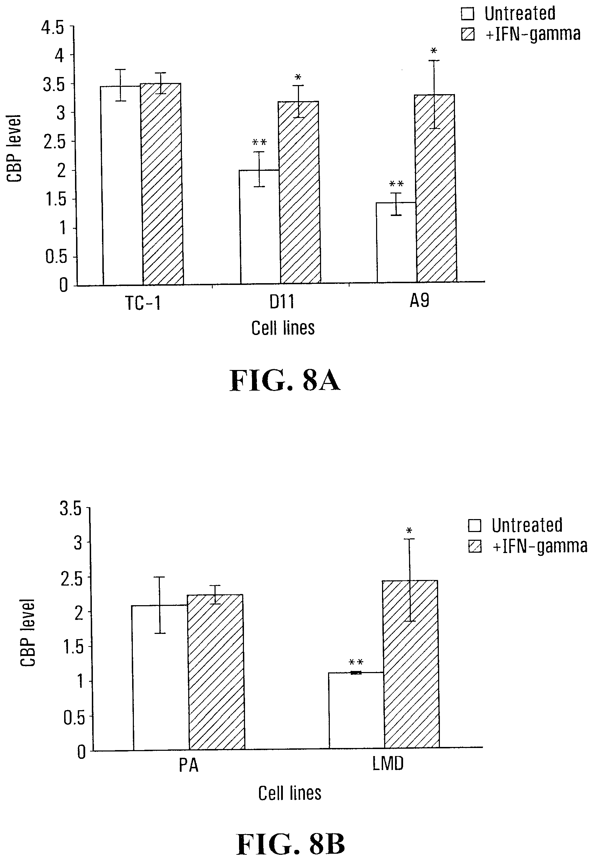

Western blot analysis showed that CBP is not deficient or truncated in TAP- deficient carcinomas (data not shown). However, chromatin immunoprecipitation analysis using primers specific for the 5'-end of the TAP-1 promoter showed that CBP binding to the region was significantly lower in TAP-deficient carcinomas (Figure 8 - CBP binding to TAP-1 promoter is lower in TAP- deficient carcinomas and is enhanced by IFN-y treatment). Chromatin immunoprecipitation using anti-CBP antibody was performed as described earlier. Columns, average of four experiments; bars, SEM. * P <.05 compared with untreated cells. ** P <.05 compared with cells that expressed the highest MHC class I in the same assay.

In addition, CBP binding to TAP-1 promoter of LMD, D11 and A9 cells were found to increase upon treatment with IFN-y (Figure 8). These results suggest that the lack of HAT activity exerted by CBP in TAP-1 promoter plays a role in the inaccessibility of the promoter by general transcription factors and RNA pol II complex, and subsequent impairment of TAP-transcription. IFN-y corrected the deficiency by improving the recruitment of factors, such as CBP, that are capable of inducing histone acetylation, thus improving the accessibility of the promoter by transcriptional machinery.

The results reported above show low RNA pol II binding to the promoter and coding region of TAP-1 gene (Figure 2), as well as low levels of activity of the promoter as the reporter construct had integrated into the genome of TAP-deficient cells (Figure 1) and indicate the presence of obstacles that limit the access of general transcription factors and RNA pol II complex to the TAP-1 promoter. A compact nucleosome structure around a promoter region appears to act as a physical barrier that prevents the binding of transcriptional activators to the promoter and consequently, halts the transcription process. One of the well-known epigenetic mechanisms that appears greatly to improve the expression of several types of genes is via the acetylation of histone H3 tails in a gene's locus, that promotes relaxation of the nucleosome structure in the region. This would in tum make the region more accessible to transcriptional machinery However, it is known that histone acetylases and deacetylases act selectively on genes, hence do not universally affect the transcription of all genes (Struhl, K, op.cit.).

In the present application, it is demonstrated that the level of histone H3 binding to TAP-1 promoter showed similar trends as the levels of RNA pol II binding to TAP 1 locus, TAP-1 expression, and surface MHC class I expression in all the cell lines tested (Figure 2). These observations indicate that the difference in histone H3 acetylation levels in TAP-i promoter plays a role in the regulation of TAP-1 transcription, although it is not likely to be the sole mechanism contributing to different levels of the gene's transcription, since the activation of transcription generally involves synergistic actions of several factors.

Treatments of TAP-deficient cells with TSA, a histone deacetylase inhibitor (HDACi), resulted in a significant increase of RNA pol II binding to TAP-1 promoter and the promoter's activity (Figure 3). In the HPV-positive carcinoma model, TSA treatment enhanced the TAP-1 promoter activity in TAP-deficient cells to similar levels as in TAP- expressing cells. In the prostate carcinoma model, although TSA treatment resulted in a statistically significant improvement of TAP-1 promoter activity in LMD cells, the degree of induction was lower than that exhibited by the D11 and A9 cells despite a high degree of induction of RNA pol II binding to the TAP-1 promoter. In addition to the previous observation that the levels of TAP-1 promoter-driven luc gene expression matched the endogenous TAP-1 expression profile equally well in both the transient and the stable transfectants (Figure 1), this suggests the presence of unknown, additional mechanisms that impair TAP-1 promoter-driven gene transcription in LMD cells.

The upregulation of TAP-1 expression observed upon treatment of the HPV- positive and prostate carcinoma cells with TSA did not occur as a result of direct increase in acetyl-histone H3 binding to the TAP-1 promoter itself (Figure 3). This suggests that TSA action involves other mechanisms than direct acetylation of histone H3 within the TAP-1 promoter region proximal to the TAP-1 gene. In addition, this may also imply that the lack of histone H3 acetylation in TAP-1 promoter is due to the lack of initial acetylation by histone acetyl transferases, instead of aberrant histone deacetylase activity that could be inhibited by TSA action.

In addition to the improvement of TAP-1 expression, treatment with TSA also resulted in the upregulation of several other APM components, such as TAP-2, LMP-2 and tapasin, and surface expression of MHC class I in TAP-deficient cells (Figure 4). This resulted in the improvement of the ability of VSV-infected cancer cells to present viral peptide in the context of K^{b}, that in turn improved the killing of the vims-infected cancer cells by VSV- specific CTLs (Figure 5A). Also, daily treatment with TSA was shown to suppress tumor growth in mice inoculated with A9 cancer cells (Figure 5B). These fmdings are encouraging for the development of therapeutic approaches that aim to increase viral or tumor antigen presentation as a way to improve the recognition and the killing of virus-infected or neoplastic cells by specific CTLs.

Despite the efficacy of TSA in improving MHC class I antigen presentation and CTL killing of the cancer cells, the levels of induction resulted from TSA treatment were always not as strong as the effects generated by IFN-y (Figures 4-6). Our results demonstrated that one of the key differences that resulted in differential ability of the two substances in improving the MHC class I antigen presentation is the difference in their ability to enhance the level of histone H3 acetylation in TAP-1 promoter. IFN-y was able to enhance histone H3 acetylation in TAP-1 promoter of TAP-deficient cells to much higher levels than TSA did (Figures 3 and 6), up to similar or even higher levels of acetyl-histone H3 in TAP-1 promoter of TAP- expressing cells. IFN-y treatment could potentially result in a maximum state of relaxation of nucleosomal structures around the TAP-1 locus, thus enabling sufficient levels of general transcription factors and RNA pol II binding to the TAP-1 promoter and efficient transcriptional activity of the gene.

Furthermore, we found that the region in TAP-1 promoter that is responsible for differential activity of the promoter in the TAP-expressing and the

TAP- deficient prostate carcinoma cells is in-between base #-557 and approximately #-401 of the 5'-end region of the promoter (Figure 7), where a CREB protein binding motif is located (Figure 7A). We found that the levels of CBP binding to this region of the TAP-i promoter in TAP- deficient cells were lower than in cells that expressed higher TAP-1 (Figure 8), indicating that the lack of histone acetyl transferase activity exerted by the well-known transcriptional co- activator plays a role in the TAP-1 deficiency in the cancer cells.

IFN-y treatment was found to enhance the association of CBP to TAP-1 promoter in TAP-deficient cells up to similar levels as in TAP-expressing cells (Figure 8). This effect may result from IFN-y-induced association of STAT-Ia and CBP (see Ma, Z., et.al., J. Leukoc Biol 78, 515 - 523) that modulates the TAP-1 promoter activity upon association of the factors with the promoter. Alternatively, IFN-y may also act through STAT-1 independent pathways, such as through a novel IFN-y -activated transcriptional element or through immediate early proteins and transcription factors (see Ramana, C.V. et.al, Trends Immol. 23, 96 - 101). By interacting simultaneously with the basal transcription machinery and with one or more upstream transcription factors, CBP may function as a physical bridge that stabilizes the transcription complex.

Results presented herein show that the lack of histone H3 acetylation in TAP-1 promoter is at least partially responsible for the deficiency in antigen processing ability and immune escape mechanisms of cancer cells.

### REFERENCES

1. Lin, K.Y., Guarnieri, F.G., Staveley-O'Carroll, K.F., Levitsky, H.I., August, J.T., Pardoll, D.M., and Wu, T.C. (1996). Treatment of established tumors with a novel vaccine that enhances major histocompatibility class II presentation of tumor antigen. Cancer Res 56, 21-26.
2. Smahel, M., Sima, P., Ludvikova, V., Marinov, I., Pokoma, D., and Vonka, V. (2003). Immunisation with modified HPV16 E7 genes against mouse oncogenic TC-1 cell sublines with downregulated expression of MHC class I molecules. Vaccine 21, 1125- 1136.
3. Lee, H.M., Timme, T.L., and Thompson, T.C. (2000). Resistance to lysis by cytotoxic T cells: a dominant effect in metastatic mouse prostate cancer cells. Cancer Res 60, 1927- 1933.
4. Franks, L.M., Carbonell, A.W., Hemmings, V.J., and Riddle, P.N. (1976). Metastasizing tumors from serum-supplemented and serum-free cell lines from a C57BL mouse lung tumor. Cancer Res 36, 1049-1055.
5. Poste, G., Doll, J., Hart, I.R., and Fidler, I.J. (1980). In vitro selection of murine B16 melanoma variants with enhanced tissue-invasive properties. Cancer Res 40, 1636-1644.
6. Gabathuler, R., Reid, G., Kolaitis, G., Driscoll, J., and Jefferies, W.A. (1994). Comparison of cell lines deficient in antigen presentation reveals a functional role for TAP-1 alone in antigen processing. J Exp Med 180,1415-1425
7. Giraud, S., Bienvenu, F., Avril, S., Gascan, H., Heery, D.M., and Coqueret, O. (2002). Functional interaction of STAT3 transcription factor with the coactivator NcoA/SRCIa. J Biol Chem 277, 8004-8011.
8. Setiadi, A.F., David, M.D., Chen, S.S., Hiscott, J., and Jefferies, W.A. (2005). Identification of mechanisms underlying transporter associated with antigen processing deficiency in metastatic murine carcinomas. Cancer Res 65, 7485-7492.
9. Berger, S.L. (2002). Histone modifications in transcriptional regulation. Curr Opin Genet Dev 12, 142-148.
10. Eberharter, A., and Becker, P.B. (2002). Histone acetylation: a switch between repressive and pennissive chromatin. Second in review series on chromatin dynamics. EMBO Rep 3,224-229.
11. Mahadevan, L.C., Clayton, A.L., Hazzalin, C.A., and Thomson, S. (2004). Phosphorylation and acetylation of histone H3 at inducible genes: two controversies revisited. Novartis Found Symp 259, 102-111; discussion 111-104, 163-109.

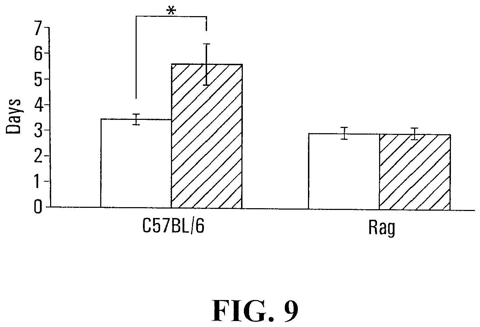

**Figure 9****. provides evidence that TSA treatment in vivo suppresses growth of tumors derived from TAP-deficient cells in C57B1/6 mice but not in immundeficient Rag1^{-/-} mice,** demonstrating that the anti-tumour effect of histone deacetylase inhibitors is mediated by an immune response not by an effect on inhibiting tumor growth or promoting apaoptosis of the tumour. A9 tumour growth was suppressed in C57B1/6 mice treated daily for 20 days with 500 [mu]g/kg of TSA (black bars) as compared to those treated with DMSO vehicle control (white bars). However, TSA treatment had no affect on A9 tumour growth in Rag1 ^{-/-} mice.

In selected embodiments the invention provides an assay for a cell treatment regimen that elicits in a cell a histone deacetylase inhibitor activity and does not elicit an apoptosis promoting activity. The assay may involve exposing a cell to one or more test compounds at one or more test concentrations; and, assaying for the level of histone deacetylase activity in the cell and assaying for the level of apoptotic activity in the cell. Apoptotic activity may for example be measured using an annexin V assay (such as the ApoAlert Annexin V assay sold by Clonetec) or an assay for the activity or inhibition of purified caspase enzymes (such as caspase -3/7 enzymes). Apoptosis assays may measure characteristics that reflect the translocation of phosphatidylserine (PS; 1-3) from the inner (cytoplasmic) leaflet of the plasma membrane to the outer (cell surface) leaflet, which takes place soon after the induction of apoptosis (annexin V protein has a strong, specific affinity for phosphatidylserine, so that labeled annexin V binding provides the basis for such an assay).

## Claims

1. Pharmaceutical composition for use in the treatment of a mammal suffering from a disorder involving excess TAP-1 expression-deficient malignant cells which are selected from the group consisting of lung carcinoma cells, prostate carcinoma cells and cervical carcinoma cells, the composition comprising a TAP-1 expression increasing amount of a histone H3 deacetylase inhibitor that promotes transcription of a TAP-1 gene in the cells to cause enhanced MHC Class I surface expression of the cells, and a suitable adjuvant or carrier.

2. The composition for use according to claim 1, wherein the malignant cells are carcinoma cells that express the papilloma E6 and E7 genes.

3. The composition for use according to claim 1 or 2, wherein the histone H3 deacetylase inhibitor is a hydroxamic acid-based histone H3 deacetylase inhibitor.

## Patentansprüche

1. Pharmazeutische Zusammensetzung zur Verwendung bei der Behandlung eines Säugers, der an einer Störung leidet aufgrund eines Überschusses an TAP-1-expressionsdefizienten malignen Zellen, welche ausgewählt sind aus der Gruppe bestehend aus Lungenkarzinomzellen, Prostatakarzinomzellen und Zervixkarzinomzellen, die Zusammensetzung umfassend eine TAP-1 Expression erhöhende Menge eines Histon-H3-Deacetylaseinhibitors, welcher Transkription eines TAP-1 Gens in den Zellen fördert, um erhöhte MHC-Klasse-I Oberflächenexpression der Zellen zu bewirken, und ein geeignetes Adjuvans oder einen Träger.

2. Zusammensetzung zur Verwendung nach Anspruch 1, wobei die malignen Zellen Karzinomzellen sind, die die Papillomagene E6 und E7 exprimieren.

3. Zusammensetzung zur Verwendung nach Anspruch 1 oder 2, wobei der Histon-H3-Deacetylaseinhibitor ein Hydroxamsäure-basierter Histon-H3-Deacetylaseinhibitor ist.

## Revendications

1. Composition pharmaceutique pour utilisation dans le traitement d'un mammifère souffrant d'un trouble associé à un excès de cellules malignes présentant une expression déficiente de TAP-1 qui sont choisies dans le groupe constitué des cellules de carcinome du poumon, des cellules de carcinome de la prostate et des cellules de carcinome du col de l'utérus, la composition comprenant une quantité augmentant l'expression de TAP-1 d'un inhibiteur d'histone H3 désacétylase qui favorise la transcription d'un gène TAP-1 dans les cellules en causant une augmentation de l'expression en surface des complexes majeurs d'histocompatibilité (MHC) de classe 1 des cellules, et un adjuvant ou un vecteur adéquat.

2. Composition pour utilisation selon la revendication 1, dans laquelle les cellules malignes sont des cellules de carcinome qui expriment les gènes de papillome E6 et E7.

3. Composition pour utilisation selon la revendication 1 ou 2, dans laquelle l'inhibiteur d'histone H3 désacétylase est un inhibiteur d'histone H3 désacétylase à base d'acide hydroxamique.
